# FASCICULE DE BREVET EUROPEEN

(11) **EP 0 507 645 B1**
(45) Date de publication et mention de la délivrance du brevet: **11.07.2001**
(21) Numéro de dépôt: 92400736.2
(22) Date de dépôt: 19.03.1992
(51) Int. Cl.: A61F 2/30

(54) **Prothèse médicale pour articulation**
Gelenkendoprothese
Joint endoprosthesis

(30) Priorité: 03.04.1991 FR 9104008
(43) Date de publication de la demande: 07.10.1992
(73) Titulaire: Bouvet, Jean-Claude, F-91590 La Ferte Alais (FR)
(72) Inventeur: Bouvet, Jean-Claude, F-91590 La Ferte Alais (FR)
(74) Mandataire: Flavenot, Bernard

(56) Documents cités:
- EP-A- 0 277 282
- WO-A-86/01394
- DE-A- 2 501 080
- US-A- 3 864 758
- US-A- 4 772 287
- US-A- 4 919 668

## Description

La présente invention concerne les prothèses médicales pour articulations, c'est-à-dire les implants utilisés pour remplacer, dans un corps vivant tel qu'un corps humain, tout ou partie de l'un ou des deux éléments mâle et femelle constituant une articulation.

Il existe de nombreuses prothèses utilisées pour remplacer des articulations d'un être vivant, par exemple l'articulation du fémur et de l'os iliaque, celle du genou, de l'épaule, etc.

En effet, lorsqu'une articulation est détériorée, il est en général nécessaire de remplacer au moins l'une des deux portions d'os et de cartilage qui coopéraient en rotation l'une dans l'autre, de façon à restituer tous les mouvements relatifs des deux os.

Si l'on prend pour exemple l'articulation entre le fémur et l'os iliaque, même si les dégradations subies par les deux parties de la rotule sont relativement mineures, il est généralement nécessaire de remplacer la tête du fémur par une sphère artificielle, par exemple en métal, céramique, monocristal, etc., et de remodeler le réceptacle cotyloïdien du bassin en y implantant une cupule artificielle qui affecte la forme d'une cuvette hémisphérique complémentaire de la sphère remplaçant la tête du fémur.

Au cours des mouvements effectués par le patient dans lequel a été implantée une telle prothèse, des frottements se produisent entre les deux parties de la prothèse, qui génèrent des particules d'usure solides du ou des matériaux qui la constituent.

Ces particules infiniment petites migrent dans la prothèse et dans ses alentours en étant considérées, par le corps vivant dans lequel elle a été implantée, commes des corps étrangers qui doivent être éliminés. Cette élimination échoit normalement aux cellules dites "macrophages". Or, la nature des matériaux constituant ces particules d'usure ne permet pas à ces particules d'être "digérées" par les cellules macrophages qui s'accumulent alors de plus en plus nombreuses autour d'elles, jusqu'à former, entre la prothèse et les tissus vivants, des agrégats fortement nuisibles au maintien durable de la prothèse.

Des prothèses ont été réalisées pour tenter de remédier aux inconvénients mentionnés ci-dessus, par exemple celles décrites dans le DE-A-2 501 080 et le US-A-3 864 758 qui comportent une enveloppe étanche réalisée en un matériau souple et un fluide incompressible contenu dans l'enveloppe.

Cependant, ces réalisations ne permettent pas de remédier aux inconvénients mentionnés auparavant car, du fait de la structure de l'enveloppe, il se produit toujours un glissement de la paroi de l'enveloppe par rapport aux deux os de l'articulation, glissement qui génère des particules d'usure dont le caractère néfaste a été explicité ci-avant.

La présente invention a donc pour but de réaliser une prothèse médicale qui permette essentiellement de réduire au maximum la production de particules d'usure dans les prothèses totales, mais aussi de conserver au moins l'une des parties de l'articulation opérée, dans le cas où les os la constituant n'auraient subi que des détériorations mineures et seraient donc dans un étant permettant une intervention chirurgicale plus légère qu'une implantation de prothèse totale.

Plus précisément, la présente invention a pour objet une prothèse permettant de restituer à une articulation le mouvement relatif de ses deux parties l'une par rapport à l'autre, comportant une enveloppe étanche réalisée en un matériau souple et un fluide incompressible emplissant ladite enveloppe, cette enveloppe étant apte à être interposée entre les deux dites parties de l'articulation devant se déplacer l'une par rapport à l'autre et au contact des deux parties, caractérisée par le fait que ledit matériau souple est un matériau non élastique.

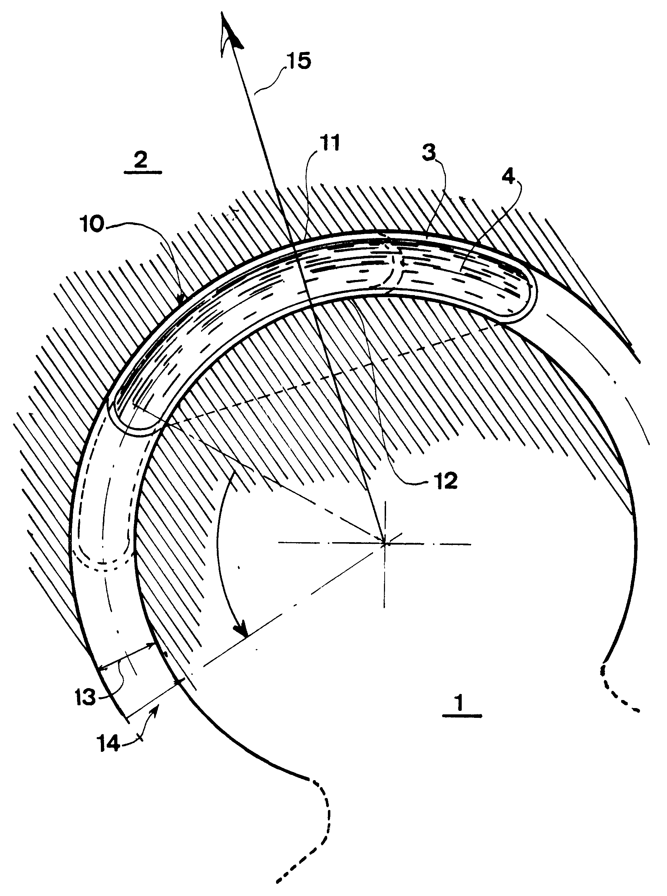

D'autres caractéristiques et avantages de la présente invention apparaîtront au cours de la description suivante donnée en regard du dessin annexé à titre illustratif, mais nullement limitatif, dans lequel:

Le figure unique représente, vu en coupe, un mode de réalisation d'une prothèse selon l'invention, dans une application à la restauration d'une articulation coxo-fémorale, bien qu'elle puisse s'appliquer à de nombreuses autres articulations de tout type.

La prothèse 10 selon l'invention permet de restituer à une articulation le mouvement relatif de ses deux parties l'une par rapport à l'autre, par exemple la tête d'un fémur 1 dans la cavité cotyloïdienne de l'os iliaque 2.

Cette prothèse comporte une enveloppe étanche 3 réalisée en un matériau souple non élastique et un fluide incompressible 4 emplissant cette enveloppe. Le fluide peut être de tout type, essentiellement liquide, par exemple du sérum physiologique, et le matériau souple est avantageusement du polyéthylène ou analogue.

Cependant, dans le but de renforcer l'étanchéité de l'enveloppe et donc d'allonger la durée de vie de la prothèse, il peut être avantageux de réaliser la paroi de l'enveloppe par un empilement d'au moins deux couches de composition différente, par exemple une couche extérieure en polyéthylène comme décrit ci-dessus et une couche intérieure en polychlorure de vinyle ou analogue.

Ainsi constituée, la prothèse 10 est interposée entre les deux parties de l'articulation devant se déplacer l'une par rapport à l'autre, de façon qu'une portion importante de l'enveloppe 3 épouse les surfaces 11, 12 des deux os en regard l'une de l'autre. Sous l'action des forces exercées sur les deux os par les muscles et tendons les reliant, l'enveloppe 3 contenant le fluide incompressible 4 se trouve pincée entre les deux surfaces 11, 12 et retenue à leur contact. Réciproquement, elle permet de maintenir les deux parties 1 et 2 de l'articulation à une distance constante 13 l'une de l'autre et, aux deux os, de se déplacer l'un par rapport à l'autre dans les mêmes mouvements que ceux d'une articulation en bon état, mais sans friction des surfaces en contact.

La prothèse médicale décrite ci-dessus s'utilise et fonctionne en effet de la façon suivante décrite dans son application à la restauration d'une articulation coxo-fémorale.

Il est tout d'abord rappelé que, quelle que soit l'importance de la détérioration des os d'une telle articulation, l'opération pratiquée actuellement pour implanter une prothèse de hanche consiste, d'une part, à enlever la tête du fémur et la remplacer par une tête sphérique artificielle constituant la partie mâle de la prothèse et, d'autre part, à remodeler la cavité cotyloïdienne de l'os iliaque pour y placer une cupule constituant la partie femelle de la prothèse.

Avec la prothèse selon l'invention, si les surfaces des os en contact, soit de la tête du fémur, soit de la cavité cotyloïdienne, ne sont pas trop abîmées, il suffit par exemple de réduire sensiblement le rayon de la tête du fémur pour aménager entre les deux os 1 et 2 un espace 14 d'épaisseur 13 et ensuite d'introduire dans cet espace la prothèse, c'est-à-dire l'enveloppe 3 remplie de son fluide incompressible 4. On s'aperçoit donc que cette opération est beaucoup moins lourde qu'une implantation de prothèse totale, ce qui n'est pas le moindre des avantages de la présente invention.

Bien entendu, une telle prothèse peut aussi s'implanter en combinaison avec une prothèse totale de hanche selon l'art antérieur, en prévoyant initialement l'aménagement de l'espace 14. Il suffit par exemple de procéder, sur la prothèse de l'art antérieur devant être implantée, à une réduction du rayon de la tête mâle, ou une augmentation de celui de la cupule, ou une combinaison des deux, et d'introduire dans cet espace la prothèse selon l'invention comme décrit ci-dessus.

Quel que soit le cas d'implantation de la prothèse selon l'invention, l'enveloppe 3 est interposée et retenue entre les portions de surfaces en regard l'une de l'autre, des parties mâle et femelle de la rotule originelle ou artificielle, en étant sensiblement centrée sur la direction 15 de la résultante des efforts habituellement et normalement exercés sur ces deux parties de l'articulation, par exemple par le fémur sur l'os iliaque.

L'enveloppe emplie de fluide imcompressible constitue en quelque sorte un coussin qui, lorsqu'il est positionné dans l'espace 14, ne se déforme pas suivant la direction radiale des parties sphériques mâle et femelle 1, 2 et prend la forme d'une calotte sphérique épaisse automatiquement centrée sur les centres confondus de ces deux parties.

Les dimensions de l'enveloppe 3 sont déterminées en fonction de la courbure et de l'aire des portions de surfaces 11, 12 des deux parties mâle et femelle 1, 2 de l'articulation coopérant entre elles.

Le Demandeur a déterminé par exemple que, dans l'application de la prothèse à la restauration d'une articulation coxo-fémorale, les dimensions de l'enveloppe sont avantageusement telles que la calotte sphérique formée soit inscrite dans un angle solide d'environ 120 degrés.

Dans ce cas, lorsque le patient sur lequel a été implantée une telle prothèse vient à se déplacer, la partie 1 de l'articulation, en l'occurrence la tête du fémur originelle ou artificielle, subit une rotation par rapport à la partie femelle 2.

Au cours du mouvement, les points de l'enveloppe 3 en contact avec les portions de surfaces 11, 12 des deux parties de l'articulation ne subissent aucun glissement par rapport à ces surfaces.

Par contre, chaque point de la partie mâle 1 entraîne dans son mouvement de rotation le point de la paroi de l'enveloppe en contact avec lui, ce qui produit un déroulement de l'enveloppe dans le même sens que cette rotation, mais dans une amplitude angulaire égale à la moitié de celle de la rotation de la partie mâle.

En effet, dans cette rotation, l'extrémité de l'enveloppe qui se déplace avec la partie mâle se décolle de la surface de cette partie pour venir se plaquer contre la surface de la partie femelle, tandis que, simultanément, l'extrémité opposée de l'enveloppe se déroule de façon inverse, sa paroi se décollant du fond de la cavité pour se plaquer contre la surface de la sphère.

Comme mentionné ci-dessus, l'enveloppe est en un matériau non élastique et le fluide est incompressible.

Cependant, aucun liquide n'est pratiquement jamais totalement incompressible et tout matériau présente toujours une légère élasticité. De ce fait, en même temps qu'elle permet la rotation de l'une des parties de l'articulation par rapport à l'autre, sans friction des surfaces en contact comme démontré ci-dessus, la prothèse selon l'invention permet en outre d'amortir les chocs transmis au fémur lors des mouvements de marche. Elle procure ainsi au patient dans lequel elle a été implantée un meilleur confort que les prothèses selon l'art antérieur.

## Revendications

1. Prothèse permettant de restituer à une articulation le mouvement relatif de ses deux parties (1, 2) l'une par rapport à l'autre, comportant une enveloppe étanche (3) réalisée en un matériau souple et un fluide incompressible (4) emplissant ladite enveloppe, cette enveloppe étant apte à être interposée entre les deux dites parties de l'articulation devant se déplacer l'une par rapport à l'autre et au contact des deux parties, caractérisée par le fait que ledit matériau souple est un matériau non élastique.

2. Prothèse selon la revendication 1, caractérisée par le fait que le fluide incompressible est du sérum physiologique.

3. Prothèse selon l'une des revendications 1 et 2, caractérisée par le fait que le matériau souple dans lequel est réalisée ladite enveloppe est du polyéthylène.

4. Prothèse selon l'une des revendications 1 et 2, caractérisée par le fait que la paroi de ladite enveloppe est constituée par un empliment d'au moins deux couches de composition différente.

## Patentansprüche

1. Prothese, die es ermöglicht, einem Gelenk die relative Bewegung seiner beiden Teile (1, 2) zu einander wieder zu verleihen, mit einem dichten Mantel (3), bestehend aus biegsamem Werkstoff, und einer nicht komprimierbaren Flüssigkeit (4), die besagten Mantel füllt, wobei der Mantel dazu geeignet ist, zwischen die beiden Gelenkteile gelegt zu werden, die sich eines in bezug zum anderen verschieben müssen, und der dabei die beiden Teile berührt, dadurch gekennzeichnet, dass besagter biegsamer Werkstoff nicht elastisch ist.

2. Prothese gemäß dem Anspruch 1, dadurch gekennzeichnet, dass die nicht komprimierbare Flüssigkeit eine physiologische Serum ist.

3. Prothese gemäß einem der Ansprüche 1 und 2, dadurch gekennzeichnet, dass der biegsame Werkstoff, aus dem besagter Mantel hergestellt wird, ein Polyethylen ist.

4. Prothese gemäß einem der Ansprüche 1 und 2, dadurch gekennzeichnet, dass die Wand besagten Mantels aus einer Stapelung aus mindestens zwei Schichten verschiedener Zusammensetzung besteht.

## Claims

1. An implant making it possible to restore, on an articulation, the relative movement of its two parts (1, 2), one in relation to the other, comprising a sealed bag (3) made of a supple material and an incompressible fluid (4) filling the said bag, this bag being designed to be placed between the two parts of the articulation that are to move one in relation to the other and in contact with both parts, characterised by the fact that the said supple material is a non-elastic material.

2. An implant according to claim 1, characterised by the fact that the incompressible fluid is a physiological serum.

3. An implant according to one of the claims 1 and 2, characterised by the fact that the supple material, from which the said bag is made, is polyethylene.

4. An implant according to one of the claims 1 and 2, characterised by the fact that two layers, placed one on top of the other and each of different composition, form the wall of the said bag.
